# EUROPÄISCHE PATENTSCHRIFT

(11) **EP 0 566 810 B1**
(45) Veröffentlichungstag und Bekanntmachung des Hinweises auf die Patenterteilung: **14.08.1996**
(21) Anmeldenummer: 92810288.8
(22) Anmeldetag: 21.04.1992
(51) Int. Cl.: A61F 2/44

(54) **Künstlicher Bandscheibenkörper**
Artificial spinal disc
Disque intervertébral artificiel

(43) Veröffentlichungstag der Anmeldung: 27.10.1993
(73) Patentinhaber: SULZER Medizinaltechnik AG, CH-8404 Winterthur (CH)
(72) Erfinder: Baumgartner, Walter, CH-9500 Wil (CH)
(74) Vertreter: Hammer, Bruno, Dr.

(56) Entgegenhaltungen:
- EP-A- 0 317 972
- EP-A- 0 346 129
- DE-A- 2 804 936
- DE-A- 3 023 353
- FR-A- 2 636 227
- US-A- 4 309 777

## Beschreibung

Die Erfindung betrifft einen künstlichen Bandscheibenkörper mit einem oberen und einem unteren Träger, welche im angrenzenden oberen bzw. unteren Wirbel verankert sind und mit einem elastischen Zwischenstück zwischen oberem und unterem Träger, wobei der obere Träger mit dem unteren Träger über das elastische Zwischenstück fest verbunden ist. Derartige Bandscheibenkörper sind z.B. aus der EP-A-0 317 972 bekannt. Dieser Bandscheibenkörper wird im Zentrum der angrenzenden Wirbelkörper implantiert. Um die notwendige Beweglichkeit der Wirbelsäule bzw. der Wirbelkörper gegeneinander aufrecht zu erhalten, ist dieser Bandscheibenkörper notwendigerweise wesentlich kleiner als der Querschnitt der angrenzenden Wirbelkörper. Dadurch wird aber die Belastung im druckempfindlichen Zentrum der Wirbelkörper so gross, dass diese geschädigt werden und dass der Bandscheibenkörper mit der Zeit immer tiefer in den Wirbelkörper einsinkt. Damit wird aber auch dessen Funktion immer stärker beeinträchtigt, und es können zusätzliche Schäden auftreten. Als weiterer wesentlicher Nachteil kann dieser bekannte Bandscheibenkörper nur schlecht auf die besonderen Gegebenheiten des Patienten wie Gewicht, Zustand der Knochensubstanz und Lage der zu ersetzenden Bandscheibe in der Wirbelsäule angepasst werden. D.h. dieser künstliche Bandscheibenkörper passt schon anfangs nicht sehr gut und wird immer schlechter.

Aufgabe der vorliegenden Erfindung ist es daher, diese bekannten Nachteile zu überwinden und einen Bandscheibenkörper zu schaffen, welcher keine Schädigungen der Wirbelkörper hervorruft, welcher sich möglichst gut den anatomischen Gegebenheiten des Patienten anpassen lässt, so dass die Beweglichkeit der Wirbelsäule an dieser Stelle wieder hergestellt wird und dass diese langfristig erhalten bleibt.

Diese Aufgabe wird erfindungsgemäss gelöst mit einem künstlichen Bandscheibenkörper nach Anspruch 1. Durch das als separater Einschubteil ausgebildete elastische Zwischenstück, welches von aussen in die Endlage einbringbar und dort fixierbar ist, wird es möglich, diesen Einschubteil genau auf die gegebene Situation bezüglich Dimensionierung und elastischer Eigenschaften auszuwählen und anzupassen. Es wäre sogar möglich, intraoperativ verschiedene auswechselbare Einschubteile auszuprobieren. Die feste Verbindung vom oberen Träger mit dem unteren Träger über das elastische Zwischenstück verhindert zudem langfristig jede schädliche Verschiebung des Bandscheibenkörpers zwischen den angrenzenden Wirbeln.

Die abhängigen Ansprüche beschreiben vorteilhafte Weiterbildungen der Erfindung. Das Zwischenstück kann einen elastischen Kunststoff aufweisen, welcher einfach und günstig herzustellen ist. Zwecks ausgeglichener niedriger Belastung der Wirbelkörper kann die Trägeroberfläche der Form der angrenzenden Wirbelkörperoberfläche angepasst sein, und die Träger können als Schalen ausgebildet sein, welche im wesentlichen dem Querschnitt der angrenzenden Wirbelkörper entsprechen. Diese Schalen können einen verstärkten Randteil aufweisen und der Randteil kann einen bewegungslimitierenden Anschlag bilden. Dies ergibt besonders günstige, den angrenzenden Wirbeln angepasste Lastübertragungen. Sehr günstige Eigenschaften können auch mit einem Anschlag erreicht werden, welcher sowohl in Höhenrichtung als auch in dazu orthogonalen Richtungen bewegungslimitierend ausgebildet ist. In einer besonders einfachen und sicheren Ausführung kann der Einschubteil in der Endlage formschlüssig mit dem oberen und unteren Träger verbunden sein z.B. mittels einer Profilierung zum Einschieben in die Träger.

Ein einfach herstellbarer Einschubteil kann einen elastomeren Tragkörper aufweisen, welcher mit einer oberen und einer unteren steifen Deckplatte fest verbunden ist, wobei der Tragkörper mit den Deckplatten formschlüssig verbunden sein kann. Geeignete Deckplatten können aus einer Titanlegierung oder einem steifen Kunststoff mit Faserverstärkung bestehen. Geeignete Tragkörper können aus gespritztem oder gegossenem Elastomer beispielsweise aus Polyurethan bestehen. Eine weitere Verbesserung der elastischen Eigenschaften kann ein ein- oder mehrlagiges Verstärkungsband um den Tragkörper herum ergeben.

Ein Schnappverschluss ergibt eine besonders einfache Fixierung des Einschubteils in seiner Endlage. Der Einschubteil kann im Sinne eines auswechselbaren Baukastenteils mit wählbarer Höhe, Winkel und elastischen Eigenschaften dem Patienten anpassbar sein. Mit Vorteil können die elastischen Eigenschaften eine erste weiche Zone, anschliessend eine mittlere Zone und letztlich eine dritte harte Zone bezüglich Deformation aufweisen. Dabei kann die weiche Zone hauptsächlich durch den elastischen Tragkörper, die mittlere Zone durch ein umgebendes Verstärkungsband und die harte Zone durch einen Anschlag des Trägerschalenrandteils bestimmt sein. Der auswechselbare Einschubteil ermöglicht auch eine einfache spätere Revision wenn die anatomischen Gegebenheiten sich nach Jahren verändert haben. Dann kann ein entsprechend angepasster neuer Einschubteil in die Träger eingebracht werden, ohne die mit den angrenzenden Wirbeln verwachsenen Träger lösen oder herausnehmen zu müssen.

Im folgenden wird die Erfindung anhand von Beispielen und Figuren weiter erläutert. Es zeigt:
- Fig. 1a, b: einen erfindungsgemässen Bandscheibenkörper mit seitlicher Einschieberichtung in zwei Ansichten;
- Fig. 2a, b: ein Beispiel mit ventraler Einschieberichtung und einem Fixierstück;
- Fig. 3: einen Einschubteil mit Schnappverschluss;
- Fig. 4: eine weitere Anschlagsform eines verstärkten Schalenrandes;
- Fig. 5: eine Verbindung von Träger mit angrenzendem Wirbelkörper;
- Fig. 6: elastische Eigenschaften mit verschiedenen Weichheitszonen.

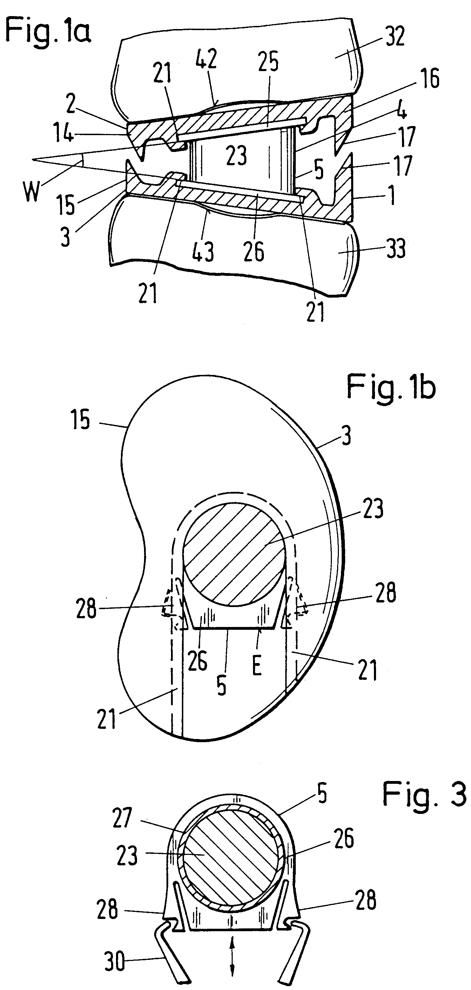

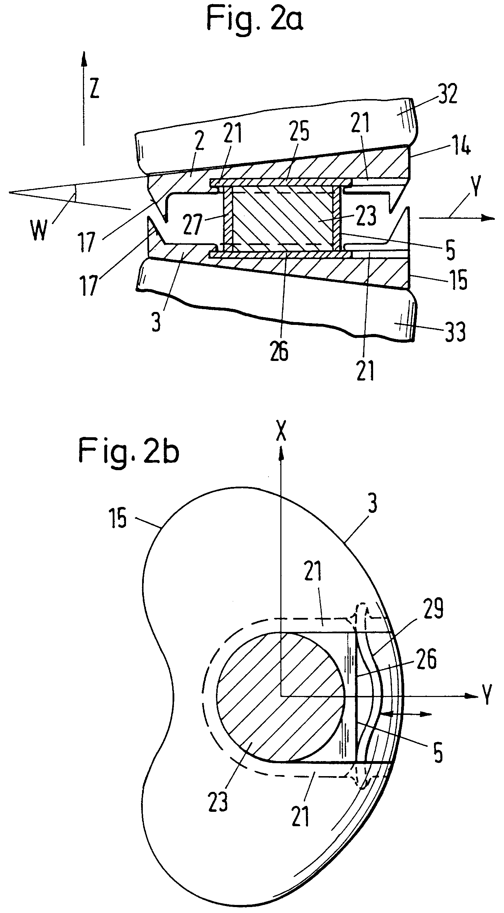

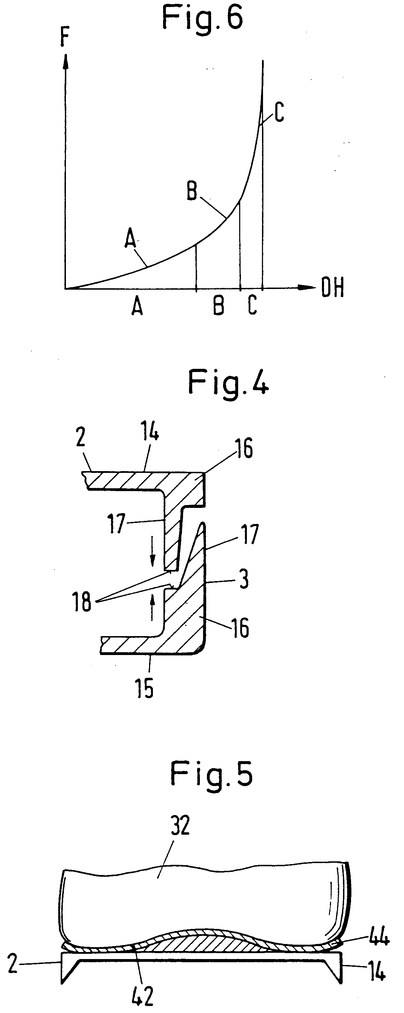

Der Bandscheibenkörper 1 von Fig. 1 weist einen oberen und unteren Träger 2, 3 auf, welche mit den angrenzenden oberen und unteren Wirbeln 32, 33 fest verbunden sind. Ein elastisches Zwischenstück 4 ist als separater Einschubteil 5 ausgebildet. Dieser ist aus seitlicher Richtung parallel zwischen die Wirbel 32, 33 einschiebbar. Dabei bilden Nuten der Träger 2, 3 und vorstehende Ränder der Deckplatten 25, 26 des Einschubteils 5 zusammen die formschlüssige Profilierung 21. In der Endlage E d.h. im Zentrum der Wirbelkörper 32, 33 wird der Einschubteil 5 an den Trägern 2, 3 fixiert, hier durch einen Schnappverschluss 28. Die Träger 2, 3 sind als Schalen 14, 15 ausgebildet, welche sich über den ganzen Wirbelquerschnitt erstrecken und damit eine gute Lastverteilung und -übertragung ergeben. Die Schalen weisen verstärkte Randteile 16 auf, welche als Anschläge 17 eine Begrenzung oder Beschränkung der relativen Bewegung der Träger 2, 3 und damit der Wirbel 32, 33 bilden. Im Beispiel von Fig. 1 weist der Bandscheibenkörper einen der Krümmung der Wirbelsäule entsprechenden Winkel W von z.B. 0° bis 20° auf, welchen obere und untere Trägerschale 14, 15 und auch die Deckplatten 25, 26 einschliessen. Ein Winkel W = 0 entspricht einer geraden Stelle der Wirbelsäule. Im Beispiel von Fig.2 sind die Deckplatten 25, 26 des Einschubteils 5 parallel angeordnet. Der Einschubteil 5 kann dann ventral in die parallelen Profilführungen 21 eingeschoben und in seiner Endlage E durch eine separate Klemmfeder als Fixierfeder 29 sicher und fest gehalten werden. Die Trägerschalen 14, 15 weisen wieder einen der Wirbelsäulenkrümmung entsprechenden Winkel W auf. Die Klemmfeder 29 kann wie auch der Schnappverschluss 28 im Beispiel von Fig.3 durch eine Spezialzange 30 bzw. ein Spannwerkzeug und wieder gelöst werden. Damit wird der Einschubteil 5 auswechselbar. Es sind aber auch andere Fixierungen z.B. durch Verschraubung möglich.

Der Einschubteil 5 besteht hier aus einem zentralen zylinderförmigen, elastomeren Tragkörper 23, welcher mit beidseitigen Deckplatten 25, 26 fest verbunden ist, z.B. chemisch oder formschlüssig durch Hinterschneidungen. Die Deckplatten können z.B. aus einer Titanlegierung oder faserverstärkten Kunststoffen bestehen. Der Tragkörper besteht beispielsweise aus elastomerem Kunststoff wie duroplastischem oder thermoplastischem Polyurethan, welches in der gewünschten Form zwischen die Deckplatten 25, 26 vergossen oder gespritzt werden kann. Ein den Tragkörper 23 umgebendes Verstärkungsband 27 z.B. aus ein- oder mehrlagigem PET dient der weiteren Optimierung der elastischen Eigenschaften des Bandscheibenkörpers.

Ein weiteres Beispiel eines Anschlags 17 des verstärkten Schalenrandteils 16 zeigt Fig. 4. Der Rand ist im oberen Teil 14 fast zylindrisch und im unteren Teil 15 konisch ausgebildet, so dass der obere Rand beim Zusammenpressen auf dem konischen unteren Rand zentriert wird bis zum Zusammenstossen der beiden Anschlagebenen 18.

Die elastischen Eigenschaften eines solchen Bandscheibenkörpers sind in Fig. 6 dargestellt. Die auftretende Kraft F in Funktion des Zusammendrückens DH des Bandscheibenkörpers weist hier drei unterschiedliche Zonen A, B, C auf. Die erste weiche Zone A wird dabei im wesentlichen durch Form und Härte des elastomeren Tragkörpers 23 bestimmt. Die folgende mittlere Zone B wird im wesentlichen zusätzlich durch das Verstärkungsband 27 und die harte Zone C durch Ausgestaltung des Schalenrandteils 16 mit Anschlag 17 bestimmt.

Die Charakteristiken A, B, C und die Dimensionierung des Bandscheibenkörpers mit Höhe H, Winkel W und Trägerschalenform und -fläche werden der gegebenen Situation des Patienten angepasst.

Eine Verankerung des Trägers 2 mit dem angrenzenden Wirbel 32 zeigt Fig. 5. Die Trägerschale 2 ist mit einem der Wirbeloberfläche 42 angepassten Metallgitter 44 z.B. Sulmesh-Titandrahtgitter, versehen, in welches Knochengewebe des Wirbels einwächst und damit eine sehr gute und dauerhafte Verbindung zum Bandscheibenkörper herstellt. Dank dem erfindungsgemässen Aufbau ist es möglich, falls nötig auch später einen allfällig geänderten, neuen Einschubteil in die gleichen festgewachsenen Trägerschalen einzubringen. Damit kann z.B. auch altersbedingten Veränderungen der Wirbelsäule Rechnung getragen werden.

### Bezeichnungsliste

- 1: Bandscheibenkörper
- 2: oberer Träger
- 3: unterer Träger
- 4: Zwischenstück
- 5: Einschubteil
- 14, 15: Schalen als 2, 3
- 16: verstärkter Randteil
- 17: Anschlag von 16
- 18: Anschlagebenen
- 21: Profilierung
- 23: Tragkörper
- 25, 26: Deckplatten
- 27: Verstärkungsband
- 28: Schnappverschluss
- 29: Klemmfeder, Fixierstück
- 30: Zange
- 32, 33: Wirbelkörper
- 42, 43: Wirbeloberfläche
- 44: Metallgitter
- E: Endlage von 5
- DH, H: Höhe von 23
- A, B, C: Elastizitätszonen
- F: Kraft
- W: Winkel
- Z, X, Y: Raumrichtungen

## Patentansprüche

1. Künstlicher Bandscheibenkörper (1) mit einem oberen (2) und einem unteren (3) Träger, welche im angrenzenden oberen bzw. unteren Wirbel (32, 33) verankerbar sind und einem elastischen Zwischenstück (4) zwischen oberem und unterem Träger, und wobei der obere Träger (2) mit dem unteren Träger (3) über das elastische Zwischenstück (4) fest verbunden ist, dadurch gekennzeichnet, dass das elastische Zwischenstück als separater Einschubteil (5) ausgebildet ist, welcher von aussen in die Endlage (E) einbringbar und dort fixierbar ist.

2. Künstlicher Bandscheibenkörper nach Anspruch 1, dadurch gekennzeichnet, dass das Zwischenstück einen elastischen Kunststoff aufweist.

3. Bandscheibenkörper nach Anspruch 1 oder 2, dadurch gekennzeichnet, dass die Trägeroberfläche (12, 13) der Form der angrenzenden Wirbelkörperoberfläche (42, 43) angepasst ist.

4. Bandscheibenkörper nach einem der vorangehenden Ansprüche, dadurch gekennzeichnet, dass die Träger als Schalen (14, 15) ausgebildet sind, welche im wesentlichen dem Querschnitt der angrenzenden Wirbelkörper (32, 33) entsprechen.

5. Bandscheibenkörper nach Anspruch 4, dadurch gekennzeichnet, dass die Schalen einen verstärkten Randteil (16) aufweisen.

6. Bandscheibenkörper nach Anspruch 5, dadurch gekennzeichnet, dass der Randteil einen bewegungslimitierenden Anschlag (17) bildet.

7. Bandscheibenkörper nach Anspruch 6, dadurch gekennzeichnet, dass der Anschlag (17) sowohl in Höhenrichtung Z als auch in den dazu orthogonalen Richtungen X, Y bewegungslimitierend ausgebildet ist.

8. Bandscheibenkörper nach einem der vorangehenden Ansprüche, dadurch gekennzeichnet, dass der Einschubteil (5) in der Endlage (E) formschlüssig mit dem oberen (2) und dem unteren Träger (3) verbunden ist.

9. Bandscheibenkörper nach einem der vorangehenden Ansprüche, dadurch gekennzeichnet, dass der Einschubteil (5) eine Profilierung (21) zum Einschieben in die Träger (2, 3) aufweist.

10. Bandscheibenkörper nach einem der vorangehenden Ansprüche, dadurch gekennzeichnet, dass der Einschubteil einen elastomeren Tragkörper (23) aufweist, welcher mit einer oberen und einer unteren steifen Deckplatte (25, 26) fest verbunden ist.

11. Bandscheibenkörper nach Anspruch 10, dadurch gekennzeichnet, dass der Tragkörper mit den Deckplatten (25, 26) formschlüssig verbunden ist (z.B. durch Hinterschneidung).

12. Bandscheibenkörper nach Anspruch 10, dadurch gekennzeichnet, dass die Deckplatten (25, 26) aus einer Titanlegierung oder einem steifen Kunststoff, z.B. mit Faserverstärkung, gebildet sind.

13. Bandscheibenkörper nach einem der Ansprüche 10 bis 12, dadurch gekennzeichnet, dass der Tragkörper (23) aus einem gespritzten oder gegossenen Elastomer wie beispielsweise Polyurethan besteht.

14. Bandscheibenkörper nach einem der Ansprüche 10 bis 13, dadurch gekennzeichnet, dass der Tragkörper (23) ein umgebendes Verstärkungsband (27), z.B. aus PET, aufweist.

15. Bandscheibenkörper nach einem der vorangehenden Ansprüche, dadurch gekennzeichnet, dass der Einschubteil durch einen Schnappverschluss (28) in seiner Endlage fixierbar ist.

16. Bandscheibenkörper nach einem der vorangehenden Ansprüche, dadurch gekennzeichnet, dass der Einschubteil (5) im Sinne eines Baukastenteils auswechselbar ist, wobei dessen Höhe H, Winkel W und elastische Eigenschaften dem Patienten angepasst wählbar sind.

17. Bandscheibenkörper nach einem der vorangehenden Ansprüche, dadurch gekennzeichnet, dass dessen elastische Eigenschaften eine erste weiche Zone A, anschliessend eine zweite mittlere Zone B und letztlich eine dritte harte Zone C bezüglich Deformation aufweisen.

18. Bandscheibenkörper nach Anspruch 17, dadurch gekennzeichnet, dass die weiche Zone A hauptsächlich durch den elastischen Tragkörper (23), die mittlere Zone B durch ein umgebendes Verstärkungsband (27) und die harte Zone C durch einen Anschlag (17) des Trägerschalenrandteils (16) bestimmt ist.

## Claims

1. Artificial intervertebral disk member (1) comprising an upper carrier (2) and a lower carrier (3) which can be anchored in the adjoining upper and lower vertebrae (32,33) respectively, and a resilient intermediate piece (4) between the upper and lower carriers, wherein the upper carrier (2) is firmly connected to the lower carrier (3) via the resilient intermediate piece (4), characterized in that the resilient intermediate piece is formed as a separate push-in insert (5) which can be introduced from the outside into the final position (E) and can be fixed there.

2. Artificial intervertebral disk member in accordance with claim 1, characterized in that the intermediate piece comprises a resilient plastic.

3. Intervertebral disk member in accordance with claim 1 or claim 2, characterized in that the carrier surface (12, 13) is matched to the shape of the adjoining surface (42, 43) of the vertebral body.

4. Intervertebral disk member in accordance with one of the preceding claims, characterized in that the carriers are formed as shells (14, 15) which essentially correspond to the cross-section of the neighbouring vertebrae (32, 33).

5. Intervertebral disk member in accordance with claim 4, characterized in that the shells have a reinforced marginal portion (16).

6. Intervertebral disk member in accordance with claim 5, characterized in that the marginal portion forms a motion limiting abutment (17).

7. Intervertebral disk member in accordance with claim 6, characterized in that the abutment (17) is made to limit motion both in the vertical direction Z and also in the directions X and Y orthogonal thereto.

8. Intervertebral disk member in accordance with one of the preceding claims, characterized in that the push-in insert (5) is connected in the end position (E) in form-fitted manner to the upper carrier (2) and to the lower carrier (3).

9. Intervertebral disk member in accordance with one of the preceding claims, characterized in that the push-in insert (5) has a profiled shape (21) for insertion into the carriers (2, 3).

10. Intervertebral disk member in accordance with one of the preceding claims, characterized in that the push-in insert has an elastomeric carrier body (23) which is fixedly connected to an upper stiff cover plate (25) and to a lower stiff cover plate (26).

11. Intervertebral disk member in accordance with claim 10, characterized in that the carrier body is connected in form-fitted manner to the cover plates (25, 26) (for example by an undercut).

12. Intervertebral disk member in accordance with claim 10, characterized in that the cover plates (25, 26) are formed of a titanium alloy or of a stiff plastic, for example with fibre reinforcement.

13. Intervertebral disk member in accordance with one of the claims 10 to 12, characterized in that the carrier body (23) comprises an injection molded or cast elastomer such as for example polyurethane.

14. Intervertebral disk member in accordance with one of the claims 10 to 13, characterized in that the carrier body (23) has a surrounding reinforcement band (27) for example of PET.

15. Intervertebral disk member in accordance with one of the preceding claims, characterized in that the push-in insert can be fixed in its end position via a snap connection (28).

16. Intervertebral disk member in accordance with one of the preceding claims, characterized in that the push-in insert (5) is interchangeable in the sense of a modular part, with the height H, angle W and resilient characteristics of the push-in insert being selectable in a manner matched to the patient.

17. Intervertebral disk member in accordance with one of the preceding claims, characterized in that its resilient characteristics have a first soft zone A, subsequent a second medium zone B and finally a third hard zone C with respect to deformation.

18. Intervertebral disk member in accordance with claim 17, characterized in that the soft zone A is principally formed by the elastic carrier body (23), the central zone B is determined by a surrounding reinforcement band (27) and the hard zone C is determined by an abutment (17) of the marginal portion (16) of the carrier shell.

## Revendications

1. Disque intervertébral (1) artificiel avec un support supérieur (2) et un support inférieur (3), qui peuvent être ancrés dans la vertèbre supérieure ou inférieure (32, 33) adjacente et avec une pièce intermédiaire (4) élastique entre le support supérieur et le support inférieur et dans lequel le support supérieur (2) est relié fixement au support inférieur (3) par la pièce intermédiaire (4) élastique, caractérisé en ce que la pièce intermédiaire élastique est configurée en élément d'insertion (5) séparé, qui peut être amené de l'extérieur dans la position de fin de course (E) et y être fixé.

2. Disque intervertébral artificiel selon la revendication 1, caractérisé en ce que la pièce intermédiaire présente une matière plastique élastique.

3. Disque intervertébral artificiel selon la revendication 1 ou 2, caractérisé en ce que la surface de support (12, 13) est adaptée à la forme de la surface de vertèbre (42, 43) adjacente.

4. Disque intervertébral artificiel selon l'une des revendications précédentes, caractérisé en ce que les supports sont des coques (14, 15), qui correspondent sensiblement à la section transversale des vertèbres (32, 33) adjacentes.

5. Disque intervertébral artificiel selon la revendication 4, caractérisé en ce que les coques présentent une partie de bordure (16) renforcée.

6. Disque intervertébral artificiel selon la revendication 5, caractérisé en ce que la partie de bordure forme une butée (17) limitant le déplacement.

7. Disque intervertébral artificiel selon la revendication 6, caractérisé en ce que la butée (17) est conçue de manière à limiter le déplacement tant dans la direction de la hauteur Z que dans les directions X, Y orthogonales à celle-ci.

8. Disque intervertébral artificiel selon l'une des revendications précédentes, caractérisé en ce que l'élément d'insertion (5) dans la position de fin de course (E) est relié par concordance de forme avec le support supérieur (2) et le support inférieur (3).

9. Disque intervertébral artificiel selon l'une des revendications précédentes, caractérisé en ce que l'élément d'insertion (5) présente un profilage (21) destiné à s'insérer dans les supports (2, 3).

10. Disque intervertébral artificiel selon l'une des revendications précédentes, caractérisé en ce que l'élément d'insertion comporte un corps de support (23) élastomère, qui est relié fixement avec une plaque de couverture rigide supérieure (25) et une plaque de couverture rigide inférieure (26).

11. Disque intervertébral artificiel selon la revendication 10, caractérisé en ce que le corps de support est relié par concordance de forme avec les plaques de couverture (25, 26) (par exemple par détalonnage).

12. Disque intervertébral artificiel selon la revendication 10, caractérisé en ce que les plaques de couverture (25, 26) sont faites d'un alliage de titane ou de matière plastique rigide, par exemple avec renfort de fibres.

13. Disque intervertébral artificiel selon l'une des revendications 10 à 12, caractérisé en ce que le corps de support (23) est fait d'un élastomère injecté ou coulé, par exemple du polyuréthanne.

14. Disque intervertébral artificiel selon l'une des revendications 10 à 13, caractérisé en ce que le corps de support (23) présente une bande de renfort (27) l'entourant, par exemple en PET.

15. Disque intervertébral artificiel selon l'une des revendications précédentes, caractérisé en ce que l'élément d'insertion peut être fixé dans sa position de fin de course, par une fermeture à déclic (28).

16. Disque intervertébral artificiel selon l'une des revendications précédentes, caractérisé en ce que l'élément d'insertion (5) est interchangeable à la manière d'un élément modulaire, sa hauteur H, son angle W et les propriétés élastiques pouvant être choisis de manière adaptée au patient.

17. Disque intervertébral artificiel selon l'une des revendications précédentes, caractérisé en ce que ses propriétés élastiques présentent une première zone A souple, puis une deuxième zone B centrale et enfin une troisième C dure en ce qui concerne la déformation.

18. Disque intervertébral artificiel selon la revendication 17, caractérisé en ce que la zone A souple est essentiellement déterminée par le corps de support (23) élastique, la zone B centrale par une bande de renfort (27) l'entourant et la zone C dure par une butée (17) de la partie de bordure (16) de la coque de support.
